# FASCICULE DE BREVET EUROPEEN

(11) **EP 2 591 447 B1**
(45) Date de publication et mention de la délivrance du brevet: **14.05.2014**
(21) Numéro de dépôt: 11744035.4
(22) Date de dépôt: 01.07.2011
(51) Int. Cl.: G06K 19/06, G06K 19/067, A61B 19/00, G03B 42/04

(54) **DISPOSITIF DE REPERAGE D'UN OBJET A RADIOGRAPHIER**
VORRICHTUNG ZUR IDENTIFIZIERUNG VON EINEM ZU RADIOGRAPHIEREN OBJEKT
DEVICE FOR IDENTIFYING AN OBJET TO BE RADIOGRAPHED

(30) Priorité: 06.07.2010 FR 1002830
(43) Date de publication de la demande: 15.05.2013
(73) Titulaire: Beweis (Sarl), 13830 Roquefort La Bedoule (FR)
(72) Inventeur: CATONIO, Jacky, F-83170 Tourves (FR); RIOUX, Mathieu, F-13821 La Penne sur Huveaune (FR); VALENSI, Jérémy, F-13530 Trets (FR); GIANGRECO, Fabrice, F-13400 Aubagne (FR)
(74) Mandataire: Roman, Alexis
(86) Numéro de dépôt international: PCT/FR2011/000381
(87) Numéro de publication internationale: WO 2012/004467

(56) Documents cités:
- FR-A1- 2 697 657
- US-A- 5 592 527

## Description

### Domaine technique de l'invention.

La présente invention concerne un dispositif permettant de relier un objet devant être radiographié au film radio sans possibilité de falsification.

### État de la technique.

On connait les dispositifs de repérage d'un objet à radiographier tels que celui décrit dans le document US 5592527 (RAY, MICHAEL A) constitué d'un corps comportant deux paires de rails à l'intérieur desquels sont agencés des symboles opaques aux ondes radios, lesdits symboles pouvant être introduits ou extraits desdites rails pour être remplacés.

On connait également les dispositifs de repérage d'un objet à radiographier tels que celui décrit dans le document FR 2697657 (DESPRES JEAN ALBERT) et constitué d'un support sur lequel on forme des symboles au moyen d'une encre opaque aux ondes radios, ladite encre n'étant pas adaptée aux supports rigides de sorte qu'elle peut aisément s'effacer.

Or, à ce jour, le repérage des objets à radiographié se fait par l'opérateur radio au moment du tir de la radio, c'est lui qui installe des numéros ou lettres en plomb sur l'objet à radiographier. Il est donc possible qu'un opérateur mal intentionné puisse modifier l'identification de l'objet. Par exemple, il met le numéro d'une soudure sur une autre soudure qu'il sait conforme.

Le dispositif selon l'invention rend impossible la falsification des clichés radios. Le but est de rendre visible sur le film de la radiographie d'un objet un identifiant que l'opérateur radio ne peut connaitre.

### Divulgation de l'invention.

La solution proposée par l'invention est un dispositif de repérage d'un objet à radiographier selon la revendication 1.

Selon une caractéristique avantageuse de l'invention, une puce RFID est noyée dans le matériau opaque et à côté de l'identifiant permettant de relier l'objet au film radiographique.

Selon une autre caractéristique avantageuse de l'invention, une protection amovible dans un matériau opaque aux rayons X et gamma peut être installée au-dessus et autour de la puce RFID afin de la protéger des ondes émises lors du tir radio.

Selon encore une autre caractéristique avantageuse de l'invention, l'identifiant inscrit sur le support détachable est lisible à l'oeil nu.

Selon encore une autre caractéristique avantageuse de l'invention, l'identifiant inscrit sur le support détachable est codé.

Selon encore une autre caractéristique avantageuse de l'invention, l'identifiant inscrit sur le support détachable est crypté.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :

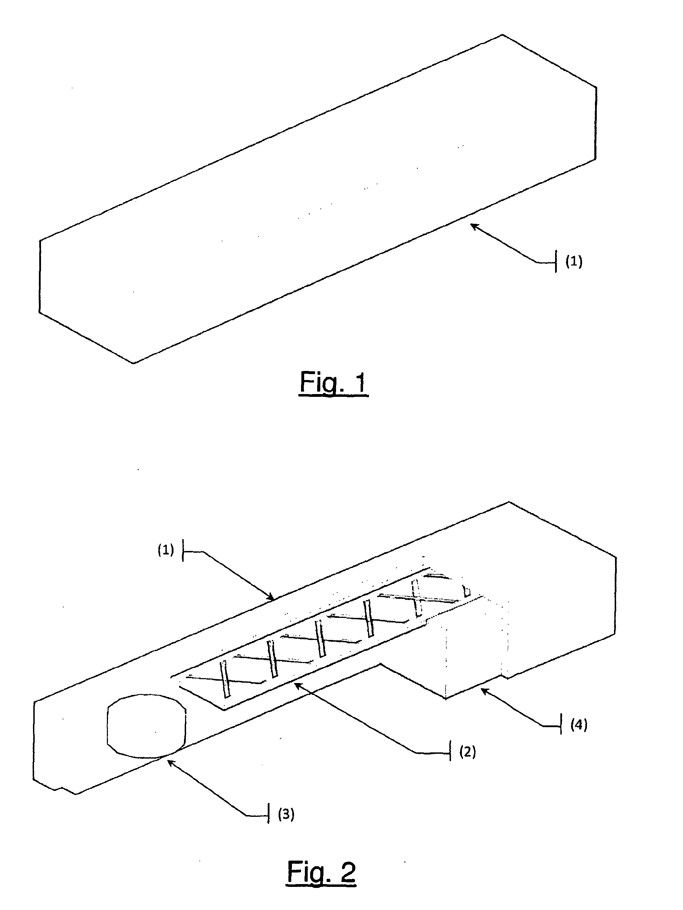

- la figure 1 représente une vue isométrique d'ensemble du dispositif de repérage.
- la figure 2 représente une vue isométrique en coupe du dispositif de repérage avec son identifiant et une puce RFID.

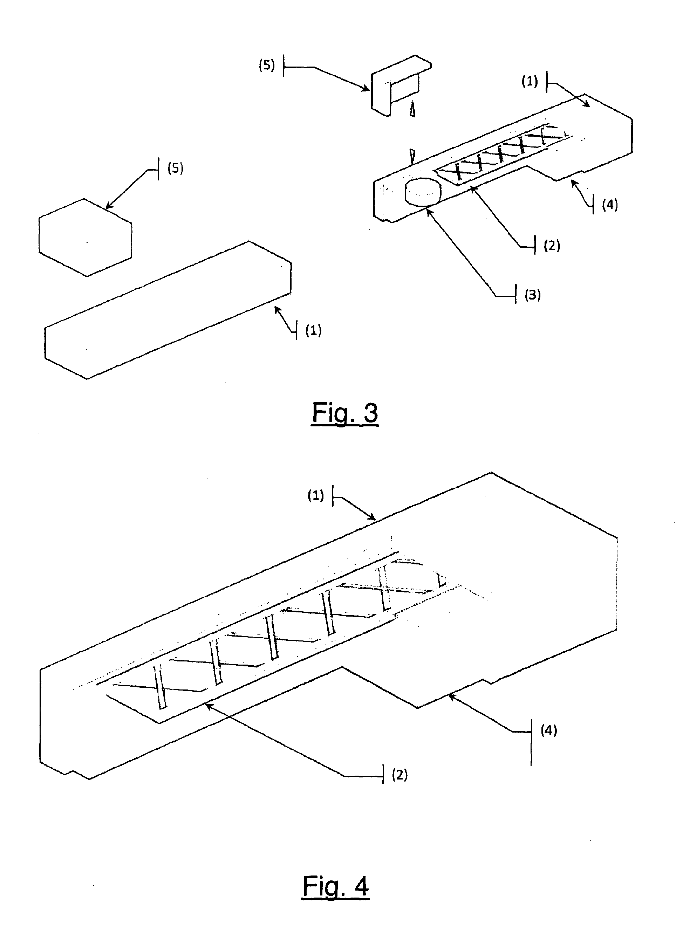

- la figure 3 représente une vue isométrique d'ensemble et en coupe du dispositif de repérage et un exemple de protection amovible isolant la puce RFID des ondes radio.
- la figure 4 représente une vue isométrique en coupe du dispositif de repérage sans puce RFID.

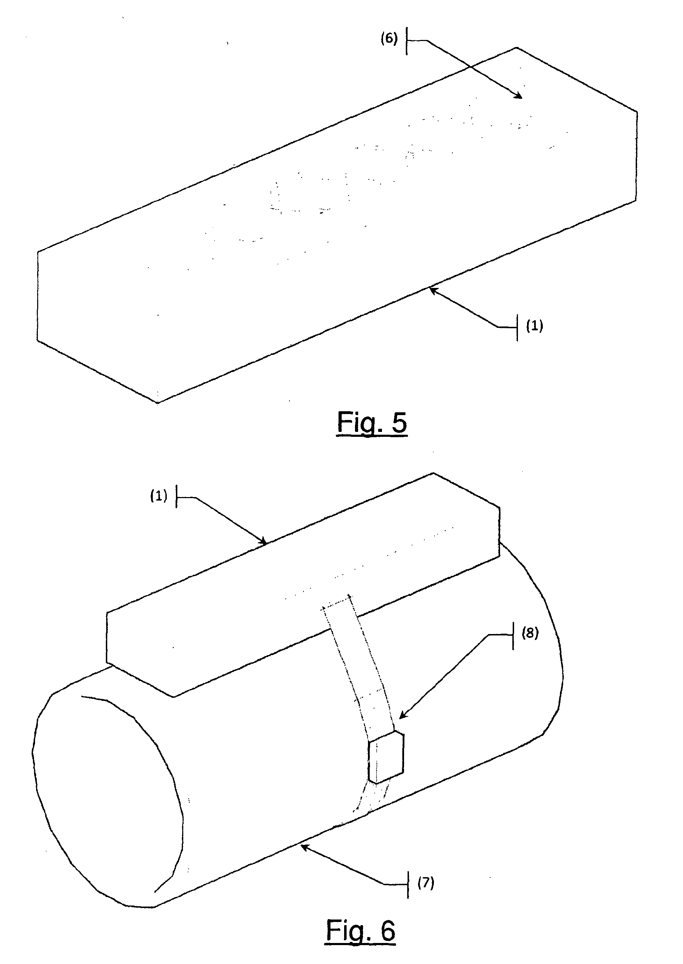

- la figure 5 représente une vue isométrique d'ensemble du dispositif de repérage avec un exemple de support détachable.
- la figure 6 représente un exemple de fixation du dispositif de repérage.

### Modes de réalisation de l'invention.

Ce dispositif comporte deux variantes que l'on peut combiner au besoin et donnant une troisième variante :
- avec une puce RFID
- avec un support détachable
- avec une puce RFID et un support détachable.

Dans le premier cas, le dispositif comporte un identifiant opaque aux rayons X, gamma ou toute autre onde servant à radiographier ainsi qu'une puce RFID qui sont coulés dans un matériau opaque (une résine opaque par exemple). L'identifiant, unique et aléatoire, est une suite de nombres, lettres et/ou symboles réalisée dans un matériau opaque au rayonnement de la source radio (en plomb par exemple). La puce RFID est positionnée à côté de l'identifiant et comporte cet identifiant dans sa mémoire. Cet identifiant peut être crypté afin qu'une lecture directe ne puisse être réalisée. Le lecteur compatible contiendra la clé de décryptage. La puce RFID peut aussi contenir d'autres informations utiles à l'objet à marquer. Ces informations peuvent être modifiables ou non, cryptées ou non selon le besoin. La puce est isolée des rayons X, gamma ou toute autre onde servant à radiographier qui pourraient l'endommagée par une protection faite dans un matériau opaque à ces ondes et placée au dessus ou autour de la puce.

Le dispositif est d'abord fixé sur l'objet par une tierce personne autre que l'opérateur radio. L'opérateur réalise le tir radio de l'objet et du dispositif de repérage. L'identifiant apparait sur le cliché radio. Ce n'est donc qu'au moment ou l'opérateur radio développe le film qu'il connait l'identifiant inscrit dans le dispositif de repérage.

Le lien entre l'objet et le cliché radio peut alors être fait en lisant le numéro de l'identifiant sur le cliché et en lisant via un lecteur compatible le numéro de l'identifiant contenu dans la puce RFID.

Dans le second cas, le dispositif comporte un identifiant qui est coulé dans un matériau opaque (une résine opaque par exemple) ainsi qu'un support détachable (qui peut être par exemple un autocollant) ou est inscrit le même identifiant aléatoire de façon codée, cryptée ou lisible à l'oeil nu. Comme dans le cas précédent, l'identifiant est une suite de nombres, lettres et/ou symboles réalisés dans un matériau opaque au rayonnement de la source radio.

Une tierce personne autre que l'opérateur radio fixe le dispositif sur l'objet à radiographier en ayant au préalable enlevé le support détachable et en l'ayant associé à l'objet (en plaçant l'autocollant à côté du nom de l'objet dans une liste par exemple). L'opérateur réalise le tir radio de l'objet et du dispositif de repérage. L'identifiant apparait sur le cliché radio. Ce n'est donc qu'au moment ou l'opérateur radio développe le film qu'il connait l'identifiant inscrit dans le dispositif de repérage.

Le lien entre l'objet et le cliché radio peut alors être fait en lisant le numéro de l'identifiant sur le cliché et en le comparant à l'identifiant inscrit sur le support détachable qui à été relié à l'objet (sur une liste par exemple).

Le troisième cas est simplement une combinaison des deux premiers et qui laisse le choix de la manière dont on relie l'objet au film radio ou qui permet de doubler ce lien.

Dans les trois cas précédents, le dispositif de repérage a été fixé à l'objet de manière que l'on puisse voir s'il a été déplacé ou retiré. Par exemple, un collier réputé inviolable solidaire du matériau opaque (la résine opaque prise en exemple ci-dessus) est serti autour de l'objet (tuyau métallique par exemple) ou à travers d'un oeilleton.

En référence aux dessins, le dispositif est constitué d'une coque (1) au fond de laquelle est positionné l'identifiant (2) et éventuellement une puce RFID (3) selon la variante choisie. L'identifiant (2) peut être une série de caractères alignés les uns à la suite des autres ou alors une plaque ajourée dans laquelle sont inscrits des caractères en négatif. L'identifiant (2) est réalisé dans un matériau opaque aux rayonnements radio, par exemple en plomb. La puce RFID (3) est surélevée du fond du boitier pour éviter des interférences avec l'objet à radiographier si celui-ci est métallique. Une résine opaque (4) est coulée dans la coque (1) afin que l'identifiant (2) et éventuellement la puce RFID (3) soient invisibles à l'oeil nu.

Une protection amovible (5) isolante des ondes radio qui peut être par exemple une sorte de couvercle est placée sur le dispositif afin de protéger la puce RFID des rayonnements radios, mais ne masque pas l'identifiant.

Le support détachable (6) présent sur la version 2 du dispositif et sur lequel est reporté l'identifiant peut être un autocollant à détacher positionné au dos du système de repérage.

Le dispositif de repérage peut être fixé sur l'objet à radiographier (7) grâce par exemple à un collier de fixation (8) avec une fermeture réputée inviolable. Ce collier est pris dans la résine opaque (4) afin d'être solidaire du dispositif.

Le dispositif selon l'invention est particulièrement destiné au repérage des soudures sur les clichés radiographiques, mais peut être utilisé sur tous les objets industriels à radiographier.

## Revendications

1. Dispositif de repérage d'un objet à radiographier constitué d'une coque (1) au fond de laquelle est positionné un identifiant alpha numérique (2) opaque aux rayons X et gamma, **caractérisé en ce qu**'une résine opaque est coulée dans la coque (1) afin que l'identifiant (2) soit invisible à l'oeil nu **et en ce que** cet identifiant est également contenu dans la mémoire d'une puce RFID (3) noyée dans la résine opaque (4) à côté de l'identifiant (2) et/ou inscrit sur un support détachable (6).

2. Dispositif de repérage selon la revendication 1 **caractérisé en ce que** l'identifiant (2) est unique et aléatoire.

3. Dispositif de repérage selon la revendication 1 **caractérisé en ce qu**'une protection amovible (5) dans un matériau opaque aux rayons X et gamma est installée au-dessus et autour de la puce RFID (3) afin de la protéger des ondes émises lors du tir radio.

4. Dispositif de repérage selon la revendication 1 **caractérisé en ce que** l'identifiant inscrit sur le support détachable (6) est lisible à l'oeil nu.

5. Dispositif de repérage selon la revendication 1 **caractérisé en ce que** l'identifiant inscrit sur le support détachable (6) est codé.

6. Dispositif de repérage selon la revendication 1 **caractérisé en ce que** l'identifiant inscrit sur le support détachable (6) est crypté.

## Patentansprüche

1. Vorrichtung zum Markieren eines Gegenstands, der geröntgt werden soll, die aus einer Schale (1) gebildet ist, auf deren Boden eine alphanumerische Kennung (2) positioniert ist, die für Röntgen- und Gammastrahlen undurchlässig ist, **dadurch gekennzeichnet, dass** ein undurchsichtiges Harz in das Gehäuse (1) gegossen ist, damit die Kennung (2) für das bloße Auge unsichtbar ist, **und dass** diese Kennung außerdem im Speicher eines RFID-Chips (3) enthalten ist, der in das undurchsichtige Harz (4) neben der Kennung (2) eingetaucht ist, und/oder in einen ablösbaren Träger (6) geschrieben ist.

2. Ortsbestimmungsvorrichtung nach Anspruch 1, **dadurch gekennzeichnet, dass** die Kennung (2) eindeutig und zufällig ist.

3. Ortsbestimmungsvorrichtung nach Anspruch 1, **dadurch gekennzeichnet, dass** über dem und um den RFID-Chip (3) ein ablösbarer Schutz (5) in einem für Röntgen- und Gammastrahlen undurchlässigen Material installiert ist, um den Chip vor Wellen, die bei einem Röntgenschuss emittiert werden, zu schützen.

4. Ortsbestimmungsvorrichtung nach Anspruch 1, **dadurch gekennzeichnet, dass** die in den ablösbaren Träger (6) geschriebene Kennung für das bloße Auge lesbar ist.

5. Ortsbestimmungsvorrichtung nach Anspruch 1, **dadurch gekennzeichnet, dass** die in den ablösbaren Träger (6) geschriebene Kennung codiert ist.

6. Ortsbestimmungsvorrichtung nach Anspruch 1, **dadurch gekennzeichnet, dass** die in den ablösbaren Träger (6) geschriebene Kennung verschlüsselt ist.

## Claims

1. Device for tagging an object to be radiographed consisting of a shell (1) in the bottom of which is positioned an alphanumeric identifier (2) opaque to X rays and gamma rays, **characterized in that** an opaque resin is cast in the shell (1) so that the identifier (2) is invisible to the naked eye **and in that** this identifier is also contained in the memory of an RFID chip (3) embedded in the opaque resin (4) alongside the identifier (2) and/or inscribed on a detachable support (6).

2. Tagging device according to Claim 1, **characterized in that** the identifier (2) is unique and random.

3. Tagging device according to Claim 1, **characterized in that** a removable protection (5) in a material opaque to X rays and gamma rays is installed above and around the RFID chip (3) so as to protect it from the waves emitted during the radio shot.

4. Tagging device according to Claim 1, **characterized in that** the identifier inscribed on the detachable support (6) is readable with the naked eye.

5. Tagging device according to Claim 1, **characterized in that** the identifier inscribed on the detachable support (6) is coded.

6. Tagging device according to Claim 1, **characterized in that** the identifier inscribed on the detachable support (6) is encrypted.
